# EUROPEAN PATENT APPLICATION

(11) **EP 4 817 505 A2**
(43) Date of publication of application: **07.10.2026**
(21) Application number: 26169537.3
(22) Date of filing: 31.03.2026
(51) Int. Cl.: A61B 1/00, A61B 1/06, A61B 1/227, A61B 1/24, A61B 3/12

(54) **IMAGE CAPTURE FOR OPTICAL VIEWING DEVICES**

(30) Priority: 31.03.2025 US 202563780805 P
(71) Applicant: Welch Allyn, INC., Skaneateles Falls, NY 13153-0220 (US)
(72) Inventor: PERKINS, David G., Skaneateles Falls, 13153-0220 (US); ENDRES, Danielle R., Skaneateles Falls, 13153-0220 (US); KRAUSE, Hongying, Skaneateles Falls, 13153-0220 (US)
(74) Representative: Reddie & Grose LLP

(57) **Abstract**

An imaging device for capturing images of an anatomy is disclosed. The imaging device includes a bracket for removably attaching the imaging device to an optical viewing device, a camera for capturing images through an eyepiece of the optical viewing device, a display screen for displaying the images captured by the camera, and a transceiver for communicating with the optical viewing device. The imaging device determines a sequence of images for capturing with the camera based on a type of the optical viewing device coupled to the imaging device. The imaging device controls an optics system of the optical viewing device to adjust properties of light projected onto the anatomy. The imaging device controls the camera to capture the sequence of images while the optics system of the optical viewing device adjusts the properties of light projected onto the anatomy.

## Description

Optical viewing devices are typically used during routine physical examinations of patients. One type of optical viewing device is an otoscope, which is used to examine the ear, including the external auditory canal and the eardrum (tympanic membrane). Otoscopes help healthcare professionals diagnose a variety of ear-related conditions. For example, an otoscope can be used to check for blockages, such as earwax buildup, foreign objects, or signs of infection in the ear canal. An otoscope can also be used to examine the eardrum such as to check for signs of infection (e.g., otitis media), perforations, scarring, or other abnormalities. Although unable to provide a direct view of the middle ear, an otoscope can help identify fluid buildup or pressure in the middle ear, which can be a sign of infection or other issues like fluid accumulation.

Another type of optical viewing device is an ophthalmoscope, which is used to examine the interior structures of the eye such as the retina, the optic nerve, and blood vessels. Ophthalmoscopes allow inspection the back of the eye for diagnosing a variety of eye conditions and systemic diseases. For example, an ophthalmoscope can provide a clear view of the retina, helping detect conditions such as retinal detachment, diabetic retinopathy, macular degeneration, and retinal hemorrhages. Also, an ophthalmoscope can be used to assess the optic nerve head for signs of optic neuritis, glaucoma, or increased intracranial pressure, which can cause changes in the appearance of the optic disc, and to visualize the blood vessels in the eye, identifying signs of high blood pressure, diabetes, or other conditions that affect blood circulation, such as retinal vein or artery occlusions. Although not a primary use, an ophthalmoscope can also assist in examining the lens and cornea for conditions like cataracts or signs of trauma.

Another type of optical viewing device is a dermatoscope, which is used to examine the skin such as to evaluate pigmented lesions, like moles and other growths. Dermatoscopes provide a magnified, illuminated view of the skin's surface, allowing for the observation of structures and patterns that are not visible to the naked eye. For example, a dermatoscope can be used to assess the color, symmetry, borders, and any irregularities in moles or skin growths, which can be important in identifying skin cancer such as melanoma. A dermatoscope can also be used for viewing pigmentation within the skin, helping to distinguish benign lesions from potentially malignant ones, and for viewing vascular structures such as tiny blood vessels such as those in basal cell carcinoma to give insight into the lesion's blood flow patterns.

In general terms, the present disclosure relates to image capture for optical viewing devices such as dermatoscopes, ophthalmoscopes, and otoscopes. In one possible configuration, an imaging device is configured for removable attachment to an optical viewing device, and controls a camera to capture a sequence of images while an optics system of the optical viewing device adjusts properties of light projected onto an anatomy. Various aspects are described in this disclosure, which include, but are not limited to, the following aspects.

One aspect relates to an imaging device for capturing images of an anatomy, the imaging device comprising: a bracket for removably attaching the imaging device to an optical viewing device; a camera for capturing images through an eyepiece of the optical viewing device; a display screen for displaying the images captured by the camera; a transceiver for communicating with the optical viewing device; and a controller communicatively coupled to the camera, the display screen, and the transceiver, the controller including a processing circuitry having a memory storing instructions which, when executed by the processing circuitry, cause the processing circuitry to: determine a type of the optical viewing device coupled to the imaging device; determine a sequence of images for capturing with the camera based at least on the type of the optical viewing device; control an optics system of the optical viewing device to adjust properties of light projected onto the anatomy; and control the camera to capture the sequence of images while the optics system of the optical viewing device adjusts the properties of light projected onto the anatomy.

Another aspect relates to a method of capturing images of an anatomy, the method comprising: determining a type of an optical viewing device coupled to an imaging device; determining a sequence of images for capturing with a camera of the imaging device based on the type of the optical viewing device; controlling an optics system of the optical viewing device to adjust properties of light projected onto the anatomy; and controlling the camera to capture the sequence of images while the optics system of the optical viewing device adjusts the properties of light projected onto the anatomy.

Another aspect relates to an imaging device for capturing images of an anatomy, the imaging device comprising: a controller including a processing circuitry having a memory storing instructions which, when executed by the processing circuitry, cause the processing circuitry to: receive a sequence of images of an anatomy, each image in the sequence of images of the anatomy having unique lighting characteristics based on one or more of wavelength, intensity, and polarization; determine a likelihood of a disease state for the anatomy based on an artificial intelligence algorithm analyzing the sequence of images; display a recommendation based on the likelihood of the disease state.

The invention will now be further described by way of example with reference to the accompanying drawings, in which:
FIG. 1 shows examples of different types of optical viewing devices, each optical viewing device is shown attached to an imaging device.
FIG. 2 is a rear isometric view of an example of an imaging device mechanically coupled to an optical viewing device of FIG. 1.
FIG. 3 is a front isometric view of the example of the imaging device of FIG. 2 positioned for mechanical coupling to the optical viewing device.
FIG. 4 schematically illustrates an example of an imaging device of FIG. 1 communicatively coupled to an optical viewing device.
FIG. 5 illustrates a rear view of an example of a head unit belonging to a dermatoscope of the optical viewing devices of FIG. 1.
FIG. 6 illustrates an example of one or more filters mounted on a wheel inside a head unit belonging to an ophthalmoscope of the optical viewing device of FIG. 1.
FIG. 7 schematically illustrates an example of a method of capturing images of an anatomy performed by an image capture application installed on the imaging device of FIG. 4.
FIG. 8 illustrates a sequence of images captured by the imaging device under different lighting conditions during performance of the method of FIG. 7.

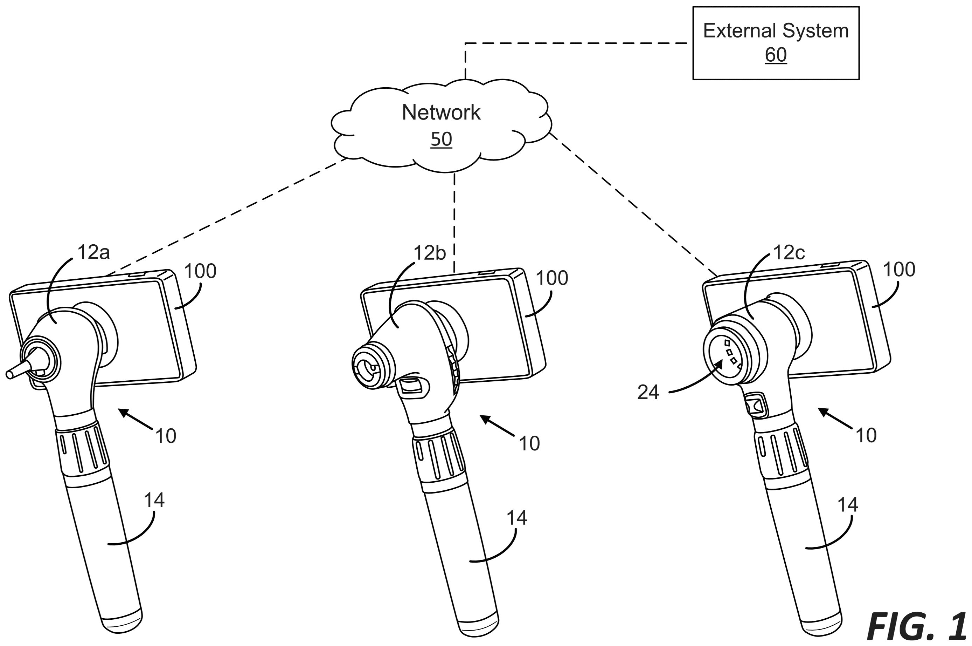

FIG. 1 shows examples of optical viewing devices 10 attached to imaging devices 100. A first type of optical viewing device 10 includes a head unit 12a of an otoscope, a second type of optical viewing device 10 includes a head unit 12b of an ophthalmoscope, and a third type of optical viewing device 10 includes a head unit 12c of a dermatoscope. Additional types of the optical viewing devices having additional types of head units are contemplated such that the present disclosure is not limited to otoscopes, ophthalmoscopes, and dermatoscopes.

As shown in FIG. 1, each type of optical viewing device 10 includes an instrument handle 14 attached to the head unit 12. The instrument handle 14 includes a power source that powers the electrical components of the head unit 12. For example, the instrument handle 14 can include rechargeable batteries, disposable batteries, or a tether to a wall transformer for supplying electrical power to the electrical components of the head unit 12.

As further shown in FIG. 1, the imaging devices 100 are mechanically coupled to the head units 12 of the optical viewing device 10. The imaging devices 100 are portable, battery powered devices that record high quality images, videos, and other data from the head units 12 of the optical viewing devices 10. Each of the imaging devices 100 captures images, videos, and other data through an eyepiece of the head unit 12 for display on a display screen. The images, videos, and other data captured by the imaging devices 100 can be analyzed by algorithms (including artificial intelligence) for disease screening. The images, videos, and other data captured by the imaging devices 100 can be stored in an electronic medical record (EMR).

The imaging devices 100 can transmit the images, videos, and other data to an external system 60, which can analyze the images, videos, and other data to generate one or more outputs for transmission back to the imaging devices 100. The imaging devices 100 can communicate with the external system 60 via a network 50. The external system 60 can be remotely located with respect to the imaging devices 100. The external system 60 can include a cloud server. In some examples, the external system 60 provides an overread service for analyzing the images, videos, and other data captured by the imaging devices 100.

The algorithms (including artificial intelligence) for disease screening can be executed on either or both of the imaging device 100 and the external system 60. In some examples, the external system 60 may also host storage of the images, videos, and other data captured by the imaging device 100. In further examples, the external system 60 can host the EMR of a patient. In yet further examples, the external system 60 may provide connectivity to other external systems and servers having image storage, or that host the EMR of the patient.

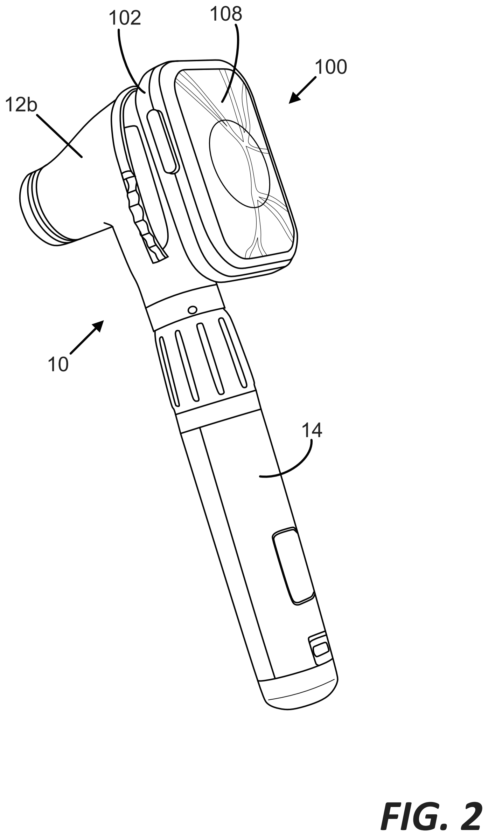

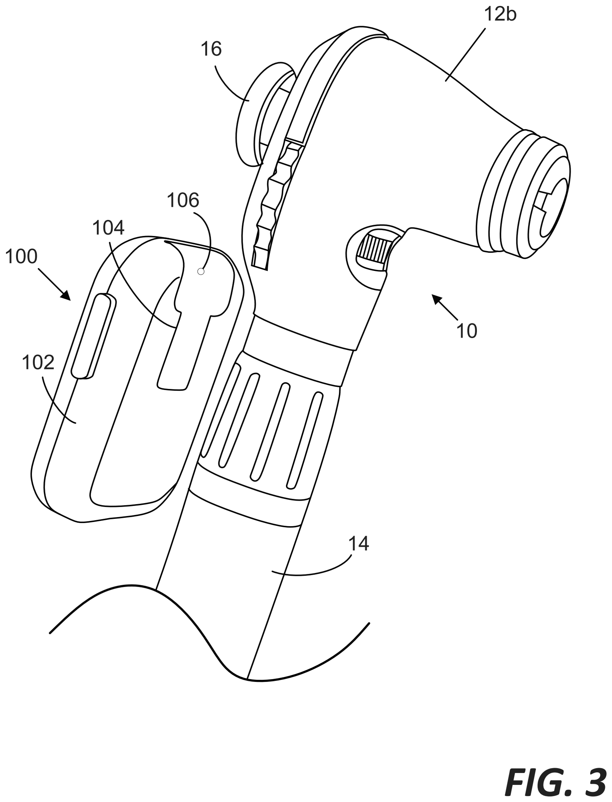

FIG. 2 is a rear isometric view of an example of the imaging device 100 mechanically coupled to an optical viewing device 10. FIG. 3 is a front isometric view of the imaging device 100 positioned for mechanical coupling to the optical viewing device 10. The imaging device 100 removably couples to the optical viewing device 10 such that the imaging device 100 can attach and detach from the optical viewing device 10 as desired. The optical viewing device 10 displays a view of an anatomy seen through an eyepiece 16 of the head unit 12b. Also, the optical viewing device 10 captures images, videos, and other data of the anatomy.

In the example of FIGS. 2 and 3, the head unit 12b belongs to an ophthalmoscope helps facilitate assessment of the health of the retina, the optic disc, and other structures of the eyes performed as part of a routine physical examination of a patient. The imaging device 100 can also attach to other types of optical viewing devices such as otoscopes for viewing inside the ears of the patient, dermatoscopes for viewing skin surfaces of the patient, and the like.

The head unit 12b includes an eyepiece 16 for viewing an anatomy. The imaging device 100 includes a housing 102 having a bracket 104 that removably attaches to the eyepiece 16. The imaging device 100 includes a camera 106 that captures images viewed through the eyepiece 16 of the head unit 12. The camera 106 is configured for alignment with the eyepiece 16 for capturing images, videos, and other data viewed through the eyepiece 16 when the imaging device 100 is attached to the head unit 12 of the optical viewing device 10.

As shown in FIG. 2, the imaging device 100 includes a display screen 108 to display the images, videos, and other data captured by the camera 106. The display screen 108 provides an area for viewing the images and videos of anatomical structures that is larger than that of the eyepiece 16 of the head unit 12. Further, the display screen 108 allows the medical professional to view the anatomical structures without having to position their face against the eyepiece 16 which improves their freedom of movement during physical examination of the patient. The display screen 108 can include a touchscreen to receive inputs from a user of the imaging device.

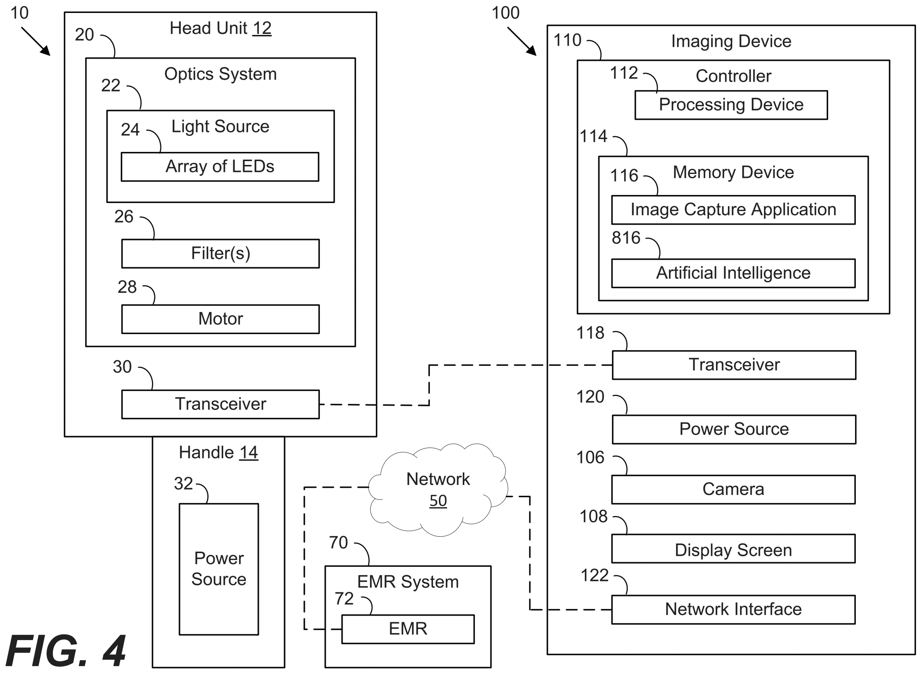

FIG. 4 schematically illustrates an example of the imaging device 100 communicatively coupled to the optical viewing device 10. As shown in FIG. 4, the head unit 12 of the optical viewing device 10 is attached the instrument handle 14. The head unit 12 includes an optics system 20 that has a light source 22 for directing light toward an anatomy such as a skin surface when the head unit 12 belongs to a dermatoscope, an eye when the head unit 12 belongs to an ophthalmoscope, or an ear when the head unit 12 belongs to an otoscope.

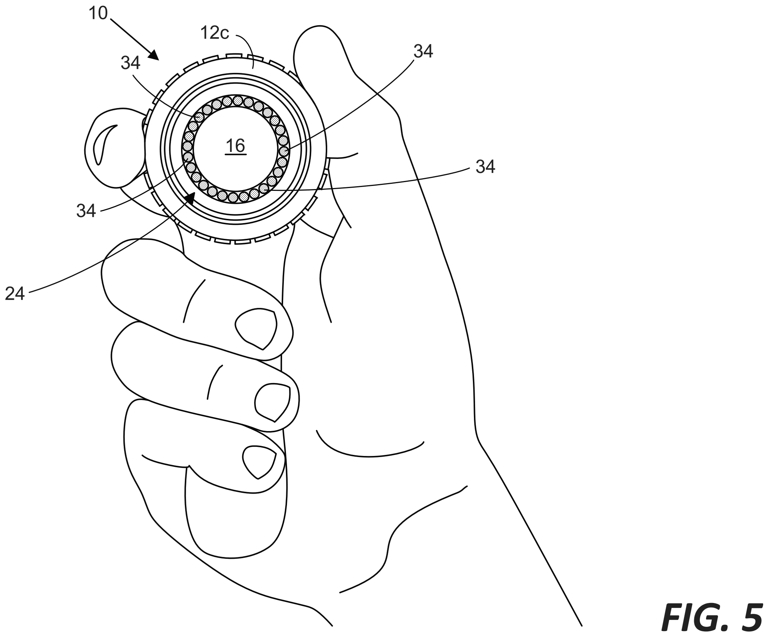

FIG. 5 illustrates a rear view of an example of a head unit 12c belonging to a dermatoscope. Referring now to FIGS. 4 and 5, the light source 22 can include an array 24 of light-emitting diodes (LEDs) 34. One or more of the LEDs 34 in the array 24 emit light having unique properties. For example, one or more of the LEDs 34 can emit nonpolarized light under different wavelengths of light such as infrared, near-infrared, visible light (i.e., bright white light, warm yellow light, and the like), and ultraviolet light. Further, one or more of the LEDs 34 in the array 24 emit polarized light such cross-polarized light, parallel polarized light, fully polarized light, partially polarized light, and the like. The imaging device 100 is programmed to control the light source 22 by turning on and off different combinations of the LEDs 34 in the array 24 to alter the properties of light projected onto an anatomy such as a skin surface, an eye, or an ear.

In the example shown in FIGS. 1 and 5, the array 24 of the LEDs 34 are shown as being arranged in a circle that surrounds a distal end of the eyepiece 16. It is contemplated that there may be alternative orientations and patterns for arranging the LEDs 34 in the array 24.

In further examples, the light source 22 can include a single source of light of varying spectra such as a tunable or broadband light source. As an illustrative example, can be achieved through methods like using a broadband light source with a tunable monochromator, or employing technologies like tunable lasers or supercontinuum sources. As an illustrative example, the light source 22 can include a halogen lamp or xenon lamp, which emits a continuous spectrum of light as a broadband light source, and a monochromator can then be used to isolate specific wavelengths for projection onto a target anatomy. As a further example, the light source 22 can include a tunable laser that emits light at a specific wavelength, and that wavelength can be adjusted or "tuned" to cover a range of wavelengths. Additional examples of the types of light sources that can be employed on the optics system 20 are contemplated.

As further shown in FIG. 4, the optics system 20 includes one or more filters 26 that can be applied to the light projected by the light source 22 to alter the properties of the light projected onto the anatomy such as the skin surface, the eye, or the ear. In such examples, the one or more filters 26 can be similar to the filters on an ophthalmoscope such as on the PanOptic^{®} Plus ophthalmoscope available from Baxter^{®}. For example, the one or more filters 26 can include a red-free filter (also known as green filter) that blocks red wavelengths of light for viewing retinal blood vessels and nerve fibers more clearly, a blue filter or cobalt blue filter for viewing corneal abrasions or ulcers with fluorescein dye, a yellow filter, and additional types of filters and polarizers. In some examples, the optics system 20 can further include an electric motor 28 that controls the positioning and/or orientation of the one or more filters 26.

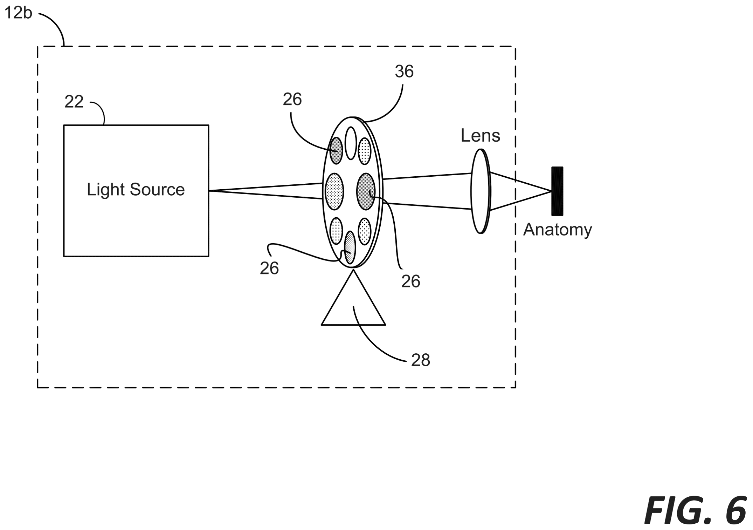

FIG. 6 illustrates an example of the one or more filters 26 mounted on a wheel 36 inside a head unit 12b belonging to an ophthalmoscope. The electric motor 28 rotates the wheel 36 to cause a filter 26 to be applied to the light projected from the light source 22, or alternatively, cause no filter to be applied to the light projected from the light source 22. The rotation of the wheel 36 by the electric motor 28 can be controlled by the imaging device 100.

Referring back to FIG. 4, the head unit 12 of the optical viewing device 10 has a transceiver 30 that communicatively couples to a transceiver 118 on the imaging device 100. In some examples, the transceivers 30, 118 are communicatively coupled only when the imaging device 100 is mechanically coupled to the optical viewing device 10. The transceivers 30, 118 can communicate via wireless protocols such as Bluetooth, near-field communication (NFC), ZigBee, ultra-wideband (UWB), Wi-Fi, and infrared (IR) data transmission. Alternatively, the transceivers 30, 118 can communicate via electrical contact communication such as when the head unit 12 includes electrical contacts that contact electrical contacts on the imaging device 100 when the imaging device 100 is mechanically coupled to the head unit 12.

The communication between the transceivers 30, 118 allows the imaging device 100 to detect attachment to the optical viewing device 10. For example, communication between the transceivers 30, 118 is not established unless the imaging device 100 is mechanically coupled to the optical viewing device 10 such that the transceiver 118 on the imaging device 100 is within a threshold distance of the transceiver 30 on the optical viewing device 10. Accordingly, the communicative coupling of the transceivers 30, 118 signals mechanical attachment of the imaging device 100 to the head unit 12 of the optical viewing device 10.

Additionally, the communication between the transceivers 30, 118 allows the imaging device 100 to determine a type of the optical viewing device 10 such as whether the optical viewing device 10 is a dermatoscope, an ophthalmoscope, or an otoscope. For example, the transceiver 30 can emit a unique signal identifying the type of the optical viewing device 10 as well as information such as a model number, version, and other relevant information.

The communication between the transceivers 30, 118 further allows the imaging device 100 to control one or more components of the optics system 20 on the head unit 12 such as to turn on and off one or more of the LEDs 34 of the light source 22, and/or to control the electric motor 28 to position the one or more filters 26 such as by rotating the wheel 36 to cause a certain filter to be applied to the light projected from the light source 22, or alternatively, to cause no filter to be applied to the light projected from the light source 22.

As shown in FIG. 4, the instrument handle 14 includes a power source 32 that powers the electrical components of the head unit 12. The power source 32 can include rechargeable batteries, disposable batteries, or a power inlet connected via a tethered power cable to a wall transformer for supplying electrical power to the electrical components of the head unit 12.

As further shown in FIG. 4, the imaging device 100 includes a controller 110 having a processing device 112 and a memory device 114. The processing device 112 and the memory device 114 can be part of a processing circuitry that executes instructions causing the processing circuitry of the imaging device 100 to perform the functions described herein.

The processing device 112 is an example of a processing unit such as a central processing unit (CPU). The processing device 112 can include one or more central processing units (CPUs). In some examples, the processing device 112 can include one or more digital signal processors, field-programmable gate arrays, or other electronic processing devices.

The memory device 114 operates to store data and instructions for execution by the processing device 112. The memory device 114 includes computer-readable media, which may include any media that can be accessed by the processing device 112. By way of illustrative example, the computer-readable media can include computer-readable storage media and computer-readable communication media. In some examples, the memory device 114 can store locally the images, videos, and other data captured by the camera 106 of the imaging device 100. Alternatively, the controller 110 can utilize cloud storage to remotely store data and software tools including artificial intelligence algorithms which are accessible over the network 50.

As shown in FIG. 5, the memory device 114 stores an image capture application 116 which controls the head unit 12 of the optical viewing device 10 (when attached to the imaging device 100) to quickly switch between a wide range of illumination patterns, such as illumination patterns having a variety of color, polarization, and intensity characteristics, such that a wide range of images of anatomies such as eyes, ears, and skin surfaces are captured. As an illustrative example, the image capture application 116 controls the optics system 20 of the optical viewing device 10 to adjust properties of light projected onto an anatomy, while simultaneously controlling the camera 106 to capture a sequence of images. The image capture application 116 instructs the camera 106 to capture the sequence of images based on the type of the head unit 12 such as whether the head unit 12 is that of a dermatoscope, an ophthalmoscope, or an otoscope.

The sequence of images captured by the image capture application 116 can be used to develop and train artificial intelligence algorithms including machine learning algorithms such as artificial neural networks (ANN) to identify various pathologies such as skin cancer, otitis media, retinal diseases, and systemic or retinal conditions. For example, the memory device 114 can further store an artificial intelligence (AI) algorithm 816 that is trained based on examples of the images captured by the image capture application 116. The Al algorithm 816 can include aspects of artificial intelligence, neural networks, machine learning, and the like. Based on the sequence of images captured by the image capture application 116, a neural network or other artificial intelligence algorithm is trained with data from the same perspective, but with different optical properties to highlight aspects of an anatomy with lighting colors, intensities, and/or polarization.

The Al algorithm 816 is executed by the imaging device 100 to analyze the sequence of images captured by execution of the image capture application 116 to generate one or more outputs such as recommended disease diagnoses. For example, depending on the anatomy that is imaged by the image capture application 116, the AI algorithm 816 can make a prediction of the likelihood of a particular disease such as skin cancer, acute otitis media, or glaucoma.

In some examples, Al algorithm 816 is executed by the imaging device 100 to detect a blink sign, which is a phenomenon that occurs when anatomical structures such as chrysalislike structures appear to blink when toggling between nonpolarized light and polarized light. The chrysalislike structures can be indicative of melanoma, which is a dangerous type of skin cancer that develops in cells that produce melanin. As another example, the blink sign occurs when anatomical structures such as comedo openings and milia cysts appear to blink when toggling between polarized light and nonpolarized light. The comedo openings and milia cysts are indicative of seborrheic keratosis, which is a common, benign (non-cancerous) skin growth. Thus, the Al algorithm 816 can be executed by the imaging device 100 to detect blink signs for distinguishing between cancerous (e.g., melanoma) and benign (non-cancerous) skin growths.

In some examples, the Al algorithm 816 includes generative artificial intelligence (GenAl) to create artificial visual media such as an artificially generated image that is based on the sequence of images captured by the image capture application 116. The artificially generated image can enhance one or more features or characteristics detected in the sequence of images, or can fill in missing gaps of information in one or more images of the sequence of images. The artificially generated image can help human operators such as users of the imaging device 100 or persons providing overread services via the external system 60 (see FIG. 1).

FIG. 4 shows the Al algorithm 816 as being stored on the memory device 114 for execution on the imaging device 100. In alternative examples, the Al algorithm 816 is stored externally such as on the external system 60 (see FIG. 1) for remote execution. In such examples, the imaging device 100 transmits via the network 50 the sequence of images to the external system 60, the external system 60 executes the Al algorithm 816 for generating one or more outputs based on the sequence of images, and the external system 60 transmits via the network 50 the one or more outputs to the imaging device 100 or to another device.

The Al algorithm 816 can be initially trained using proprietary data belonging to a manufacturer of the imaging device 100. The training architecture can incorporate multiple layers of data integration and processing. The AI algorithm 816 can learn from a large dataset of example images captured by the image capture application 116 on the imaging device 100.

The training methodology can implement sophisticated pattern recognition by processing historical data from large patient populations. This includes analyzing previous recommendations correlated to examples of images captured by the image capture application 116 on the imaging device 100. Inputs for training the AI algorithm 816 can include outputs of other AI models whether they are discrete classification or regression Al models or other types of models. The Al algorithm 816 can employ closed-loop learning, continuously improving its performance based on success rates of its recommendations. The neural network architecture for the Al algorithm 816 is designed with consideration given to various training techniques specifically chosen for medical applications. For example, the training process is deliberately controlled and targeted, recognizing the stringent requirements of medical device applications.

Contextual awareness can be built into the training process through integration of multiple healthcare data sources. For example, the AI algorithm 816 can learn to incorporate patient medical history data from electronic medical records (EMRs). This comprehensive data integration enables the AI algorithm 816 to make informed and contextually appropriate recommendations based on the images captured by the image capture application 116.

Training of the Al algorithm 816 can include an initial step of data collection and preprocessing which can include collecting a diverse dataset of anatomical images, normalizing and standardizing the images, applying data augmentation techniques to expand the training set, and labeling and segmenting relevant anatomical features in the images.

The training of the AI algorithm 816 can further include selecting a model architecture that can, for example, implement an encoder-decoder network structure, use multiple convolutional layers for feature extraction, include skip connections to preserve spatial information, and incorporate attention mechanisms to focus on relevant anatomical regions.

The training of the AI algorithm 816 can include using supervised learning or unsupervised learning. The training can utilize a loss function combining structural similarity metrics, feature-based loss terms, and anatomical consistency constraints. The training can further implement gradient descent optimization and use mini-batch training for efficiency. The training can include validation testing performed by validating against held-out test sets, measuring accuracy using standard medical imaging metrics, ensuring anatomical consistency in generated outputs, and performing cross-validation across different anatomical variations.

Lastly, the training of the Al algorithm 816 can include post-processing such as by applying quality enhancement filters, implementing anatomical constraint checking, ensuring output meets medical imaging standards, and validating spatial relationships between anatomical structures. The Al algorithm 816 can be continuously refined using feedback loops and periodic retraining to improve accuracy and reliability of the outputs generated by the algorithm.

Referring back to FIG. 4, the computer-readable storage media on the memory device 114 includes volatile and nonvolatile, removable and non-removable media implemented in any device configured to store information such as computer-readable instructions, data structures, program modules, or other data. The computer-readable storage media can include random access memory, read only memory, electrically erasable programmable read only memory, flash memory, and other memory technology including any medium used to store information for access by the processing device 112. The computer-readable storage media is non-transitory.

The computer-readable communication media embodies computer-readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer-readable communication media includes wired media such as a wired network or direct-wired connection, and wireless media such as acoustic, radio frequency, infrared, and other wireless media. Combinations of any of the above are within the scope of computer-readable media.

The imaging device 100 includes a network interface 122 for connecting the imaging device 100 to one or more networks such as the network 50. The network interface 122 allows the imaging device 100 to communicate over the network 50 with external systems and devices. For example, the network interface 122 allows the imaging device 100 to communicate over the network 50 with an electronic medical record (EMR) system 70 for transmitting the images, videos, and other data captured by the camera 106 for storage in an electronic medical record (EMR) 72 of a patient who is being examined by use of the optical viewing device 10.

The EMR system 70 maintains the medical history of a plurality of patients by consolidating health information for each patient, including patient demographics, medical diagnoses, treatment plans, medications, test results, physiological variable measurements, and immunization history into a single document (i.e., an electronic medical record (EMR)). The EMR system 70 manages a plurality of EMRs for a plurality of patients. The EMR system 70 enables sharing of data among healthcare professionals to improve coordination of care, reduce errors, and enhance patient outcomes. The EMR system 70 also supports clinical decisionmaking by providing real-time access to comprehensive patient health information. The EMR system 70 can include secure data storage and integration with other healthcare technologies, which streamline administrative tasks and improve overall healthcare efficiency.

The network interface 122 further allows the imaging device 100 to communicate over the network 50 with the external system 60 (FIG. 1) such as for overread services and/or cloud-based storage. The imaging device 100 transmits the images and other data over the network 50 using substitutable medical applications and reusable technologies (SMART) on fast healthcare interoperability resources (FIHR), or other health information transfer protocols.

As further shown in FIG. 4, the imaging device 100 includes a power source 120 for powering the various components of the imaging device 100 including the camera 106, the display screen 108, the controller 110, the transceiver 118, and the network interface 122. The power source 120 can include rechargeable batteries, disposable batteries, or the like.

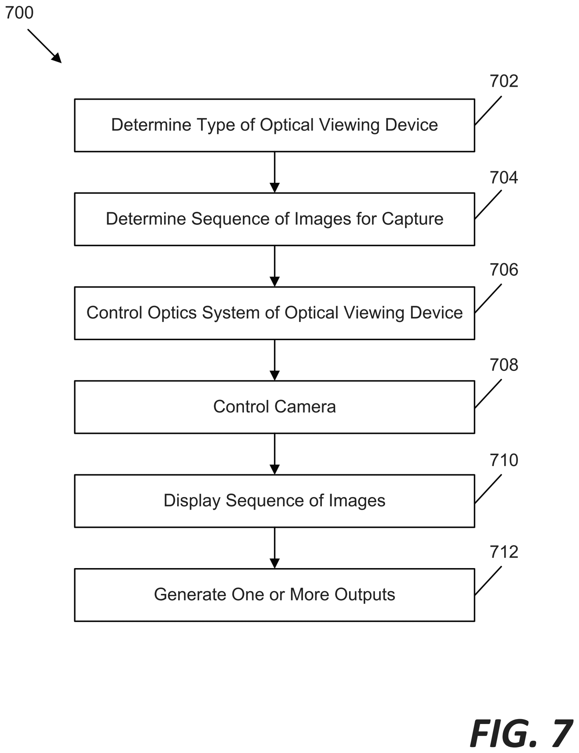

FIG. 7 schematically illustrates an example of a method 700 of capturing images of an anatomy. The method 700 can be performed by the image capture application 116 installed on the imaging device 100 to capture a sequence of images under different lighting conditions.

The method 700 includes an operation 702 of determining a type of the optical viewing device 10 coupled to the imaging device 100. As described above, the transceiver 30 on the head unit 12 of the optical viewing device 10 can emit a unique signal that is received by the transceiver 118 on the imaging device 100 for identifying the type of the optical viewing device 10. For example, the signal emitted by the transceiver 30 can identify the optical viewing device 10 as a dermatoscope, an ophthalmoscope, or an otoscope. The signal emitted by the transceiver 30 of the optical viewing device 10 can also include additional data such as a model or version of the optical viewing device 10, or an owner of the optical viewing device 10.

The method 700 can include an operation 704 of determining a sequence of images for capturing with the camera 106 of the imaging device 100. In some examples, a predetermined sequence of images is selected based on the type of the optical viewing device 10 (i.e., a dermatoscope, an ophthalmoscope, or an otoscope) determined in operation 702. A plurality of predetermined sequences of images available for selection in operation 704 may vary the types of images or quantities of images that are included in the sequence of images, and may also vary the order in which the images are captured in the sequence of images.

In further examples, the sequence of images may also vary based on one or more characteristics of the patient who is examined with the optical viewing device 10 such as the patient's age, medical history, comorbidities, diagnoses, medications, or other relevant health information that can influence the examination of the patient performed using the optical viewing device 10. Such health information can be acquired from the EMR 72 of the patient.

The method 700 includes an operation 706 of controlling the optics system 20 of the optical viewing device 10 to adjust properties of light projected onto the anatomy to capture the sequence of images determined in operation 704. Operation 706 includes using the transceiver 118 to communicate commands to the transceiver 30 on the head unit 12 to control aspects of the optics system 20 for adjusting the properties of the light projected onto the anatomy. In some examples, operation 706 can include controlling the intensity of the light projected onto the anatomy such as increasing or decreasing a quantity of lumens emitted by the light source 22.

In some examples, operation 706 can include turning on and off one or more of the LEDs 34 in the array 24 of the light source 22 to adjust the properties of the light projected onto the anatomy by the head unit 12 of the optical viewing device 10. For example, one or more LEDs 34 that project cross-polarized light can be turned on while all other LEDs 34 are turned off. As another example, one or more LEDs 34 that project parallel polarized light can be turned on while all other LEDs 34 are turn off. As another example, one or more LEDs 34 that project nonpolarized light (e.g., bright white light, warm yellow light, or ultraviolet light) can be turned on while all other LEDs 34 are turned off. Additional examples of the LEDs 34 that can be turned on and/or off for adjusting the properties of the light projected onto the anatomy are contemplated. The turning on and off of the LEDs 34 in the array 24 is synchronized with image capture by the camera 106 of the imaging device 100 to capture the sequence of images. In further examples, operation 706 can include side-lighting a skin surface by illuminating each LED 34 in sequence one-by-one around the full circle of the array 24.

Alternatively, or additionally, operation 706 can include causing a filter of the one or more filters 26 to be applied to the light emitted by the light source 22 to adjust the properties of the light projected onto the anatomy. For example, operation 706 can include sending a command that causes the electric motor 28 to rotate the wheel 36 having the one or more filters 26 to cause a certain filter to be applied to the light projected from the light source 22, or alternatively, to cause no filter to be applied to the light projected from the light source 22. Examples of the types of filters that can be applied in operation 706 can include, without limitation, a red-free filter (also known as green filter) that blocks red wavelengths of light for viewing retinal blood vessels and nerve fibers more clearly, a blue filter or cobalt blue filter for viewing corneal abrasions or ulcers with fluorescein dye, a yellow filter, or any other type of filter. The application of the filters 26 to the light from the light source 22 is synchronized with image capture by the camera 106 of the imaging device 100 to capture the sequence of images.

The method 700 includes an operation 708 of controlling the camera 106 to capture the sequence of images determined in operation 704. Operation 708 occurs simultaneously with operation 706 such that the camera 106 on the imaging device 100 captures the sequence of images while the optics system 20 on the head unit 12 of the optical viewing device 10 adjusts the properties of the light projected onto the anatomy such as a skin surface, eye, or ear canal.

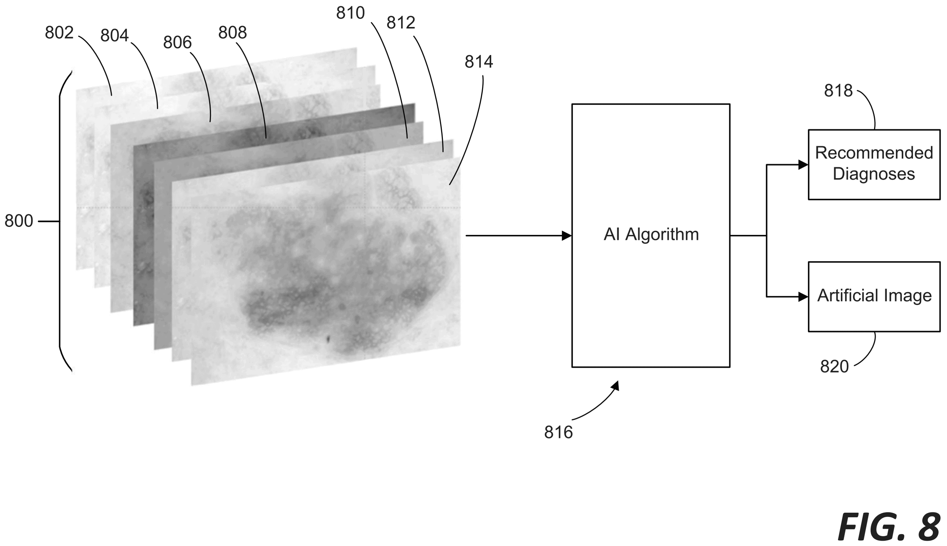

FIG. 8 illustrates an example of a sequence of images 800 that can be captured by the camera 106 of the imaging device 100 while the optics system 20 on the head unit 12 of the optical viewing device 10 adjusts the properties of the light projected onto the anatomy during performance of operations 706, 708 in the method 700. In some examples, the sequence of images 800 includes at least two images. In the illustrative example shown in FIG. 8, the sequence of images 800 includes a first image 802 captured with nonpolarized white light illumination, a second image 804 captured with nonpolarized warm light illumination, a third image 806 captured with 50% polarized light, a fourth image 808 captured with full (i.e., 100%) polarized light, a fifth image 810 captured with a cobalt-blue filter applied to the light emitted by the light source 22, a sixth image 812 captured with a red-free filter applied to the light emitted by the light source 22, and a seventh image 814 captured with ultraviolet light.

Each image in the sequence of images 800 can include metadata such as a tag that identifies the lighting conditions under which the image was captured by the camera 106 of the imaging device 100. In further examples, the tag associated with each image can identify a type of anatomy (e.g., skin surface, eye, or ear) such as based on whether the head unit 12 belongs to a dermatoscope, ophthalmoscope, or otoscope. In further examples, the tag associated with each image can identify the patient such as by including the patient's medical record number (MRN). In some examples, the imaging device 100 creates a single file that contains the sequence of images 800, with each image tagged with the metadata described above.

It is contemplated that the sequence of images 800 can include additional images captured with additional types of light properties, and/or can include fewer images than the ones shown in FIG. 8. Further, it is contemplated that the order in which the sequence of images 800 is captured may vary. For example, the types, quantities, and/or order of the images captured in the sequence of images 800 may vary based on whether the head unit 12 of the optical viewing device 10 belongs to a dermatoscope, ophthalmoscope, or otoscope. In some examples, the types, quantities, and/or order of the images captured in the sequence of images 800 may also vary based on one or more characteristics of the patient acquired from the EMR 72 of the patient.

Referring back to FIG. 7, the method 700 can include an operation 710 of displaying the sequence of images 800 on the display screen 108 of the imaging device 100. In some examples, the sequence of images 800 is displayed on the display screen 108 after completion of an exam performed using the optical viewing device 10. Each image of the sequence of images 800 can include a label identifying the properties of the light projected onto the anatomy by the head unit 12 when the image was captured by the camera 106 of the imaging device 100. In examples where the display screen 108 is a touchscreen, a user of the imaging device can scroll through the sequence of images 800 such as by swiping their finger on the touchscreen.

The method 700 includes an operation 712 of generating one or more outputs for display in addition to displaying the sequence of images. In some examples, the one or more outputs and the sequence of images are both displayed on the display screen 108 of the imaging device 100. Additionally, or alternatively, the one or more outputs and the sequence of images can both be displayed on a display screen that is separate of the imaging device 100, such as the display screen on a workstation computer, a television, or other type of peripheral display device.

As shown in FIG. 8, operation 712 can include generating one or more recommended diagnoses 818 by feeding the sequence of images 800 to the Al algorithm 816. In some examples, the Al algorithm 816 includes an artificial neural network (ANN), a deep learning network, and/or other types of machine learning models and algorithms. The Al algorithm 816 can analyze each image individually in the sequence of images 800, and the Al algorithm 816 can also analyze the sequence of images 800 collectively, to generate the recommended diagnoses 818 based on the anatomy analyzed by the optical viewing device 10.

As an illustrative example, when the imaging device 100 is coupled to an ophthalmoscope, the AI algorithm 816 can analyze the sequence of images 800 collectively to detect conditions related to the structure and function of the retina that are not ordinarily detectable using ophthalmoscopes. For example, while ophthalmoscopes can be used to identify some signs of macular edema like hard exudates or retinal thickening in the macular region, ophthalmoscopes are not considered the most accurate tool for detecting macular edema, as the detailed structural changes in the retina associated with edema are best visualized using Optical Coherence Tomography (OCT) which provides a more precise measurement of retinal thickness. In the present example, by using the Al algorithm 816 to analyze the sequence of images 800 collectively, conditions such as macular edema are detectable by the imaging device 100, which improves the effectiveness of the ophthalmoscope for detecting retinal conditions.

In some examples, operation 712 can include detecting a presence of an anatomical structure in the sequency of images 800 by detecting a blink sign when switching between a polarized image and a nonpolarized image in the sequence of images 800, or when switching between the nonpolarized image and the polarized image in the sequence of images 800. As an illustrative example, when toggling between images captured with nonpolarized light and images captured with polarized light, anatomical structures that are more conspicuous with polarized light appear to blink. Also, when toggling between images captured with polarized light and images captured with nonpolarized light, anatomical structures that are more conspicuous with nonpolarized light appear to blink. In some examples, the AI algorithm 816 is trained to detect the blink sign when switching between the images in the sequence of images 800.

The Al algorithm 816 can be trained to identify anatomical structures based on detection of the blink sign. For example, by detecting the blink sign, the AI algorithm 816 can be trained to identify anatomical structures such as chrysalislike structures which are indicative of melanoma, and comedo openings and milia cysts which are indicative of seborrheic keratosis, which is a common, benign (non-cancerous) skin growth that appears as raised, wart-like lesions.

In some examples, operation 712 can also include creating artificial visual data such as an artificial image 820 (see FIG. 8) based on the sequence of images 800. In such examples, the Al algorithm 816 includes generative Al (GenAI) for creating the artificial image 820 based on the sequence of images 800. For example, the artificial image 820 created by the GenAl combines elements from at least two images in the sequence of images 800.

As an illustrative example, when one image in the sequence of images 800 has poor quality and/or gaps of missing details or information, data from one or more other images in the sequence of images 800 is used to improve the quality of the image and/or to fill in the gaps of missing details and information. Accordingly, the artificial image 820 combines qualities, data, and other information from two or more images in the sequence of images 800. In such examples, the GenAl understands complimentary relationships between different lighting modalities used for capturing the images individually in the sequence of images 800, and the GenAl utilizes these complimentary relationships to create the artificial image 820.

In some instances, the blink sign which can result from transitioning between polarized light and nonpolarized light, or from transitioning between nonpolarized light and polarized light, can cause distortion or degraded quality of one or more images in the sequence of images 800 that are captured by the camera 106 of the imaging device 100. In such instances, the artificial image 820 created by the GenAI mitigates the distortion and/or quality degradation caused by the blink sign phenomenon. The artificial image 820 created by the GenAl improves the functioning of the imaging device 100 by mitigating distortion and/or quality degradation caused by the blink sign when transitioning between capture of polarized images and nonpolarized images, or between capture of nonpolarized images and polarized images.

The artificial image 820 that is created by the GenAI can be displayed on the display screen 108 of the imaging device 100, or on another display device communicatively coupled to the imaging device 100 such as a workstation display monitor. In some examples, the artificial image 820 can include a label that identifies the image as having been created by the GenAl. In such examples, the artificial image 820 enhances disease screening by improving qualities of the individual images in the sequence of images 800. Further, the artificial image 820 can emphasize certain features that are difficult to detect with the naked eye to enhance review of the images by a human operator such as a user of the imaging device 100 or an overread medical professional after completion of an exam performed using the optical viewing device 10.

Embodiments of the invention can be described with reference to the following numbered clauses, with preferred features laid out in the dependent clauses:
1. An imaging device for capturing images of an anatomy, the imaging device comprising:
   a bracket for removably attaching the imaging device to an optical viewing device;
   a camera for capturing images through an eyepiece of the optical viewing device;
   a display screen for displaying the images captured by the camera;
   a transceiver for communicating with the optical viewing device; and
   a controller communicatively coupled to the camera, the display screen, and the transceiver, the controller including a processing circuitry having a memory storing instructions which, when executed by the processing circuitry, cause the processing circuitry to:
      determine a type of the optical viewing device coupled to the imaging device;
      determine a sequence of images for capturing with the camera based at least on the type of the optical viewing device;
      control an optics system of the optical viewing device to adjust properties of light projected onto the anatomy; and
      control the camera to capture the sequence of images while the optics system of the optical viewing device adjusts the properties of light projected onto the anatomy.
2. The imaging device of clause 1, wherein the memory stores additional instructions which, when executed by the processing circuitry, further cause the processing circuitry to:
   display the sequence of images on the display screen, each image of the sequence of images including a label identifying the properties of light projected onto the anatomy.
3. The imaging device of clause 1 or 2, wherein the memory stores additional instructions which, when executed by the processing circuitry, further cause the processing circuitry to:
   detect presence of an anatomical structure in the sequency of images by identifying a blink sign when switching between a polarized image and a nonpolarized image in the sequence of images, or when switching between the nonpolarized image and the polarized image in the sequence of images.
4. The imaging device of any preceding clause, wherein the memory stores additional instructions which, when executed by the processing circuitry, further cause the processing circuitry to:
   create an artificial image by feeding the sequence of images to a generative artificial intelligence algorithm; and
   displaying the artificial image on the display screen.
5. The imaging device of clause 4, wherein the artificial image combines elements from at least two images in the sequence of images.
6. The imaging device of any of clauses 1-5, wherein the memory stores additional instructions which, when executed by the processing circuitry, further cause the processing circuitry to:
   turn on and off one or more light emitting diodes in an array of light emitting diodes on the optical viewing device to adjust the properties of light projected onto the anatomy.
7. The imaging device of any of clauses 1-6, wherein the memory stores additional instructions which, when executed by the processing circuitry, further cause the processing circuitry to:
   cause one or more filters to be applied to the light projected onto the anatomy to adjust the properties of light projected onto the anatomy.
8. The imaging device of any of clauses 1-7, wherein the memory stores additional instructions which, when executed by the processing circuitry, further cause the processing circuitry to:
   train an artificial intelligence algorithm using the sequence of images.
9. The imaging device of any of clauses 1-8, wherein the sequence of images is determined based on whether the optical viewing device is a dermatoscope, an ophthalmoscope, or an otoscope.
10. The imaging device of any of clauses 1-9, wherein the sequence of images includes at least two of:
   a first image captured with cross-polarized light;
   a second image captured with parallel polarized light;
   a third image captured with a cobalt-blue filter;
   a fourth image captured with a red-free filter;
   a fifth image captured with nonpolarized white light illumination;
   a sixth image captured with nonpolarized warm light illumination; and
   a seventh image captured with ultra-violet light.
11. A method of capturing images of an anatomy, the method comprising:
   determining a type of an optical viewing device coupled to an imaging device;
   determining a sequence of images for capturing with a camera of the imaging device based on the type of the optical viewing device;
   controlling an optics system of the optical viewing device to adjust properties of light projected onto the anatomy; and
   controlling the camera to capture the sequence of images while the optics system of the optical viewing device adjusts the properties of light projected onto the anatomy.
12. The method of clause 11, further comprising:
   displaying the sequence of images on the display screen, each image of the sequence of images including a label identifying the properties of light projected onto the anatomy.
13. The method of clause 11 or 12, further comprising:
   detecting presence of an anatomical structure in the sequency of images by identifying a blink sign when switching between a polarized image and a nonpolarized image in the sequence of images, or when switching between the nonpolarized image and the polarized image in the sequence of images.
14. The method of any one of clauses 11 to 13, further comprising:
   creating an artificial image by feeding the sequence of images to a generative artificial intelligence algorithm; and
   displaying the artificial image on the display screen.
15. The method of clause 14, wherein the artificial image combines elements from at least two images in the sequence of images.
16. The method of any of clauses 11-15, further comprising:
   turning on and off one or more light emitting diodes in an array of light emitting diodes on the optical viewing device to adjust the properties of light projected onto the anatomy.
17. The method of any of clauses 11-16, further comprising:
   causing one or more filters to be applied to the light projected onto the anatomy to adjust the properties of light projected onto the anatomy.
18. The method of any of clauses 11-17, further comprising:
   training an artificial intelligence algorithm using the sequence of images.
19. The method of any of clauses 11-18, wherein the sequence of images is determined based on whether the optical viewing device is a dermatoscope, an ophthalmoscope, or an otoscope.
20. The method of any of clauses 11-19, wherein the sequence of images includes at least two of:
   a first image captured with cross-polarized light;
   a second image captured with parallel polarized light;
   a third image captured with a cobalt-blue filter;
   a fourth image captured with a red-free filter;
   a fifth image captured with nonpolarized white light illumination;
   a sixth image captured with nonpolarized warm light illumination; and
   a seventh image captured with ultra-violet light.
21. An imaging device for capturing images of an anatomy, the imaging device comprising:
   a controller including a processing circuitry having a memory storing instructions which, when executed by the processing circuitry, cause the processing circuitry to:
   receive a sequence of images of an anatomy, each image in the sequence of images of the anatomy having unique lighting characteristics based on one or more of wavelength, intensity, and polarization;
   determine a likelihood of a disease state for the anatomy based on an artificial intelligence algorithm analyzing the sequence of images;
   display a recommendation based on the likelihood of the disease state.
22. The imaging device of clause 21, wherein the disease state includes skin cancer, acute otitis media, or glaucoma.
23. The imaging device of clause 21, wherein the memory stores additional instructions which, when executed by the processing circuitry, further cause the processing circuitry to:
   train the artificial intelligence algorithm using the sequence of images.
24. The imaging device of clause 21, wherein the memory stores additional instructions which, when executed by the processing circuitry, further cause the processing circuitry to:
   detect presence of an anatomical structure in the sequency of images by identifying a blink sign when switching between a polarized image and a nonpolarized image in the sequence of images, or when switching between the nonpolarized image and the polarized image in the sequence of images.
25. The imaging device of clause 21, wherein the memory stores additional instructions which, when executed by the processing circuitry, further cause the processing circuitry to:
   create an artificial image by using generative artificial intelligence that combines elements from at least two images in the sequence of images.

## Claims

1. An imaging system for capturing images of an anatomy, the imaging system comprising:
a bracket for removably attaching an imaging device to an optical viewing device;
a camera for capturing images through an eyepiece of the optical viewing device;
a display screen for displaying the images captured by the camera;
a transceiver for communicating with the optical viewing device; and
a controller communicatively coupled to the camera, the display screen, and the transceiver, the controller including a processing circuitry having a memory storing instructions which, when executed by the processing circuitry, cause the processing circuitry to:
determine a type of the optical viewing device coupled to the imaging device;
determine a sequence of images for capturing with the camera based at least on the type of the optical viewing device;
control an optics system of the optical viewing device to adjust properties of light projected onto the anatomy; and
control the camera to capture the sequence of images while the optics system of the optical viewing device adjusts the properties of light projected onto the anatomy.

2. The imaging system of claim 1, wherein the memory stores additional instructions which, when executed by the processing circuitry, further cause the processing circuitry to:
display the sequence of images on the display screen, each image of the sequence of images including a label identifying the properties of light projected onto the anatomy; and
wherein the sequence of images is determined based on whether the optical viewing device is a dermatoscope, an ophthalmoscope, or an otoscope.

3. The imaging system of claim 1 or 2, wherein the memory stores additional instructions which, when executed by the processing circuitry, further cause the processing circuitry to:
detect presence of an anatomical structure in the sequency of images by identifying a blink sign when switching between a polarized image and a nonpolarized image in the sequence of images, or when switching between the nonpolarized image and the polarized image in the sequence of images.

4. The imaging system of any one of claims 1-3, wherein the memory stores additional instructions which, when executed by the processing circuitry, further cause the processing circuitry to:
create an artificial image by feeding the sequence of images to a generative artificial intelligence algorithm, wherein the artificial image combines elements from at least two images in the sequence of images; and
displaying the artificial image on the display screen.

5. The imaging system of any one of claims 1-4, wherein the memory stores instructions which, when executed by the processing circuitry, cause the processing circuitry to perform at least one of:
turn on and off one or more light emitting diodes in an array of light emitting diodes on the optical viewing device to adjust the properties of light projected onto the anatomy; and
cause one or more filters to be applied to the light projected onto the anatomy to adjust the properties of light projected onto the anatomy.

6. The imaging system of any one of claims 1-5, wherein the memory stores additional instructions which, when executed by the processing circuitry, further cause the processing circuitry to:
train an artificial intelligence algorithm using the sequence of images.

7. The imaging system of any one of claims 1-6, wherein the sequence of images includes at least two of:
a first image captured with cross-polarized light;
a second image captured with parallel polarized light;
a third image captured with a cobalt-blue filter;
a fourth image captured with a red-free filter;
a fifth image captured with nonpolarized white light illumination;
a sixth image captured with nonpolarized warm light illumination; and
a seventh image captured with ultra-violet light.

8. A method of capturing images of an anatomy, the method comprising:
determining a type of an optical viewing device coupled to an imaging device;
determining a sequence of images for capturing with a camera of the imaging device based on the type of the optical viewing device;
controlling an optics system of the optical viewing device to adjust properties of light projected onto the anatomy; and
controlling the camera to capture the sequence of images while the optics system of the optical viewing device adjusts the properties of light projected onto the anatomy.

9. The method of claim 8, further comprising:
displaying the sequence of images on the display screen, each image of the sequence of images including a label identifying the properties of light projected onto the anatomy, wherein the sequence of images is determined based on whether the optical viewing device is a dermatoscope, an ophthalmoscope, or an otoscope.

10. The method of claim 8 or 9, further comprising:
detecting presence of an anatomical structure in the sequency of images by identifying a blink sign when switching between a polarized image and a nonpolarized image in the sequence of images, or when switching between the nonpolarized image and the polarized image in the sequence of images.

11. The method of any one of claims 8-10, further comprising:
creating an artificial image by feeding the sequence of images to a generative artificial intelligence algorithm, wherein the artificial image combines elements from at least two images in the sequence of images; and
displaying the artificial image on the display screen.

12. The method of any one of claims 8-11, further comprising at least one of:
turning on and off one or more light emitting diodes in an array of light emitting diodes on the optical viewing device to adjust the properties of light projected onto the anatomy; and
causing one or more filters to be applied to the light projected onto the anatomy to adjust the properties of light projected onto the anatomy.

13. The method of any one of claims 8-12, further comprising:
training an artificial intelligence algorithm using the sequence of images.

14. The method of any one of claims 8-13, wherein the sequence of images includes at least two of:
a first image captured with cross-polarized light;
a second image captured with parallel polarized light;
a third image captured with a cobalt-blue filter;
a fourth image captured with a red-free filter;
a fifth image captured with nonpolarized white light illumination;
a sixth image captured with nonpolarized warm light illumination; and
a seventh image captured with ultra-violet light.

15. An imaging device for capturing images of an anatomy, the imaging device comprising:
a controller including a processing circuitry having a memory storing instructions which, when executed by the processing circuitry, cause the processing circuitry to:
receive a sequence of images of an anatomy, each image in the sequence of images of the anatomy having unique lighting characteristics based on one or more of wavelength, intensity, and polarization;
determine a likelihood of a disease state for the anatomy based on an artificial intelligence algorithm analyzing the sequence of images;
display a recommendation based on the likelihood of the disease state.
